# EUROPEAN PATENT APPLICATION

(11) **EP 1 000 616 A1**
(43) Date of publication of application: **17.05.2000**
(21) Application number: 98203236.9
(22) Date of filing: 25.09.1998
(51) Int. Cl.: A61K 9/70, A61K 7/16, A61K 7/22

(54) **Prevention and treatment of parodontitis**

(71) Applicant: Schaeken, Mathias Jozef Maria, 6524 CV Nijmegen (NL)
(72) Inventor: Schaeken, Mathias Jozef Maria, 6524 CV Nijmegen (NL)
(74) Representative: Smulders, Theodorus A.H.J., Ir.

(57) **Abstract**

The present invention relates to a novel method for preventing or treating parodontitis. The method comprises treating a mammal having the disease with an antimicrobial composition comprising a physiologically acceptable varnish base and dissolved therein an antimicrobial agent.

## Description

The invention relates to a method for preventing and treating parodontitis in a mammal.

Periodontal disease is an inflammatory lesion caused by bacteria affecting the tissues housing the roots of the teeth. The disease, sometimes referred to as pyorrhea, increases in prevalence and severity with increasing age, and it is a major cause of tooth loss in humans throughout the world. When only the gum tissue or gingiva is affected, the disease is called gingivitis, but when the process extends into the deeper structures of the dental area, it is known as parodontitis. The space between the tooth and the gingival tissue that is formed when the attachment of the tooth to the bone is lost, is called a periodontal pocket.

An essential difference between the two types of periodontal disease referred to above, are the microorganisms causing it. Whereas gingivitis is caused by accumulation of the bacteria that normally occur in the microbial flora on the teeth, parodontitis finds its origin in the occurrence or overgrowth of pathogenic bacteria. Bacteria associated with the occurrence of parodontitis are, inter alia, *Prevotella intermedia, Fusobacterium nucleatum, Treponema pallidum*, *Porphyromonas gingivalis, actinobacillus actinomycetemcomitans, Bacteroides forsythus, Campilobacter rectus, Eikenella corrodens, Peptostreptococcus micros*, *Selenomonas* sp., *Eubacterium* sp., *Spirochetes* and *Streptococcus intermedius*.

The standard treatment of parodontitis consists of thorough mechanical cleaning, the so called scaling and rootplaning, of the affected (roots of the) teeth. However, when specific periodontal pathogens are present adjunctive antimicrobial treatment is necessary, and for this reason antibiotics such as tetracyclines, penicillines, metronidazole or the combination of metronidazole plus amoxycillin are prescribed. The kind of antibiotics that is to be used is determined by the composition of the pocket microflora. The antibiotics should be used always in combination with mechanical treatment in order to decrease the bacterial load in the pockets and to stimulate blood circulation.

The systemic use of antibiotics has obvious disadvantages such as impairment of the gut and vagina flora, and allergic reactions. Moreover, the increasing resistance of bacteria against antibiotics causes growing concern. For this reason local forms for the application of antimicrobial agents have been developed that do not cause these systemic side effects. Examples of these forms are for instance sustained release devices. Disadvantages of these sustained release devices are the laborious application, the need to remove or replace the device in subsequent sessions, or the inability to reach effective concentrations of the antimicrobial agent for the elimination of the periodontal pathogens.

The present invention aims to provide a novel and effective manner for preventing and treating parodontitis, which does not have the disadvantages of the prior art methods.

Surprisingly, it has been found that parodontitis may effectively be combated by use of a varnish comprising an antimicrobial agent. Thus, the invention relates to a method for treating parodontitis in a mammal, wherein said mammal is treated with an antimicrobial composition comprising a physiologically acceptable varnish base and dissolved therein an antimicrobial agent.

A significant reduction in the presence of pathogenic bacteria in periodontal pockets treated in accordance with the invention has been observed. In addition, the presence of sulphate reducing bacteria has been found to be reduced after carrying out a method according to the invention, which is a further indication that periodontal decomposition was stopped by the present treatment. Furthermore, it has been observed that the treatment of the invention significantly reduces the depth of the treated periodontal pockets. Thus, the treatment of the present invention for treating parodontitis is at least as effective as the above mentioned conventional methods of treatment, while it does not have the disadvantages of said conventional methods.

An antimicrobial composition comprising a varnish base and an antimicrobial agent is in itself known from the international patent application WO-A-89/10113. This document discloses the use of said composition for the elimination of *Streptococcus* mutans in the treatment of caries.

A method for treating parodontitis according to the invention is directed to the treatment of mammals, in particular of humans.

As mentioned above, according to the invention use is made of an antimicrobial composition comprising a physiologically acceptable varnish base and dissolved therein an antimicrobial agent. The antimicrobial composition is homogeneous and generally liquid or gel-like.

As varnish base, a resin dissolved in a solvent can be used. Both synthetic as well as natural resins can be applied. The resin and the solvent are to be chosen such that the antimicrobial agent is soluble in the varnish base. The retention of the antimicrobial composition and/or release rate of the antimicrobial agent from the antimicrobial composition to the treated organism, may be controlled by choosing a suitable varnish base. Obviously, for dental applications the varnish base must be physiologically acceptable in that it must not lead to an unacceptable degree of undesired side effects when applied in the dental orifice, e.g. on teeth, skin or mucosa. Further, the varnish base preferably does not cause negative taste perception.

Suitable resins are for example Sandarac, Siam or Sumatra benzoin, mastic, tolu balsam, or a combination thereof. Particularly, Sandarac has been found to lead to good results when it is used as resin in the varnish base. The solvent wherein the resin may be dissolved in order to form the varnish base may be ethanol, isopropanol, n-propanol, or a combination thereof. The concentration of the resin in the antimicrobial composition preferably ranges from 20% to 55%.

The antimicrobial agent in the antimicrobial composition is chosen for its activity against the pathogenic bacteria causing parodontitis. Suitable antimicrobial agents are chosen from the groups of bisbiguanides, and non-charged phenolic agents. Specific antimicrobial agents that have been found to lead to particularly good results are chlorhexidine or a salt thereof, and triclosan. Preferably, chlorhexidine or a salt thereof, such as the acetate salt, is used.

The antimicrobial agent is preferably present in an amount between 5 and 80 wt.%, more preferably between 10 and 50 wt.%, based on the weight of the antimicrobial composition. The varnish base is preferably present in an amount between 20 and 95 wt.%, preferably between 50 and 90 wt.%, based on the weight of the antimicrobial composition.

In a preferred embodiment, the antimicrobial composition further comprises additional ingredients, of which it is known that they have a beneficial effect in mouth care. Examples of such ingredients include (1) antibiotics, (2) quaternary ammonium compounds, such as cetyl pyridinium chloride, (3) plant alkaloids, such as sanguinarine, (4) metal salts, such as zinc, (5) non-charged phenolic agents, such as triclosan and (6) essential oils, (7) surface modifying agents, such as delmiponol, (8) oxidative enzymes, such as amyloglucosidases or glucose oxidases, (9) peroxides, such as hydrogen peroxide, (10) phosphate compounds, such as pyrophosphates, (11) bicarbonates, such as sodium bicarbonate, (12) (non-steroidal) anti-inflammatory agents, such as ketordac tromethamine, (S)-ketoprofen, ibuprofen, flurbiprofen, naproxen, (13) protease inhibitors, such as tetracycline analogues, Batimastat, (14) calcium lactate, calcium lacto phosphate, (poly-)calcium lactate and combinations thereof. Particularly preferred metal salts are fluorides and citrates, such as stannous fluoride or sodium fluoride and zinc citrate. Together these additional ingredients will preferably not make up more than 20 wt.% of the antimicrobial composition. The mutual amounts of these additional ingredients can be conveniently optimized by the person skilled in the art.

The antimicrobial composition is preferably applied to a periodontal pocket, i.e. at the location where the presence of the disease is apparent. As has been set forth hereinabove, a periodontal pocket is a space between gums and teeth created by the receding of the gums in a relatively advanced stage of parodontitis. It will be understood, however, that the composition may also be applied to other pockets, of which it is not clear whether it is affected by the action pathogenic bacteria.

The risk for severe parodontitis is significantly elevated in the presence of risk factors such as increasing age, tobacco smoking, diabetes mellitus or other systemic diseases, or in the presence of certain bacteria such as Porphyromonas gingivalis, Prevotella intermedia or Bacteroides forsythus. By application of the antimicrobial composition in the non-diseases gingival sulcus, parodontitis can be prevented. It is envisaged that in the latter case, the antimicrobial composition may have a prophylactic action.

Conveniently, the antimicrobial composition is applied using a syringe provided with a blunt needle that can easily be introduced in the pocket. Usually, an amount of about 250 mg of the antimicrobial composition will suffice for the treatment of the complete dentition. In practice, treatment will substantially be restricted to areas affected by pathogenic bacteria, in which case smaller amounts of the composition are sufficient.

Disease-active sites affected by periodontal pathogens can easily be detected using modern diagnostic methods such as DNA/RNA probes, or chair site tests such as immunoassays (Evalusite^{R}) or enzyme assays (Periscan^{R}, Periogard^{R}). It is to be noted that the efficacy of the present method may be increased by repetition of the method. Suitable intervals of time between subsequent treatments are between five and ten days.

Further, it has been found that parodontitis may lead to complications in medically compromised patients as pockets may serve as reservoirs for bacteria that are capable of inducing infections elsewhere in the body. The antimicrobial composition can be used as prophylaxis in the prevention of e.g. infective endocarditis, prostheitc joint infections and brain abcesses, and of infections in patients who are immunocompromised e.g. as a result of cancer therapy, are asplenic, have implanted cardiac pacemakers, suffer from diabetes, or have received various types of grafts. Thus, the invention is also directed to a method for preventing diseases associated with infections induced by bacteria in periodontal pockets in a mammal, wherein said mammal is treated with the above antimicrobial composition. For this purpose the antimicrobial composition should be applied regularly, e.g. with 3-month intervals, in the normal gingival crevice or in periodontal pockets.

The invention will now be elucidated by the following, non-restrictive examples.

### Example 1.

After informed written consent, the effect of the antimicrobial composition was investigated in a double blind clinical trial. Fourteen periodontal patients participated. In five patients 5-10 periodontal pockets were present and in eight patients even more than 10. In each subject 2 pockets harboring high levels of periodontal pathogens were selected. One pocket was treated once with the antimicrobial composition, while the other pocket served as a control and was not treated. In contrast with standard treatment of parodontitis, application of the antimicrobial composition was not supported by concomitant mechanical treatment. Analysis of the baseline bacterial composition of the periodontal pockets revealed that in the control and experimental group thorough mechanical treatment was indicated in respectively 12 and 10 pockets and that adjunctive antibiotics were indicated in five subjects. After application of the antimicrobial composition, mechanical treatment (Table 1) was significantly less indicated than in the control pockets, and, in contrast with the control group, antibiotics were not necessary anymore.

**Table 1**

| mechanical treatment necessary | control | antimicrobial composition |
|---|---|---|
| before | 12 | 10 |
| after 1 month | 7 | 2 * |

| | | |
|---|---|---|
| * P<.05 | | |

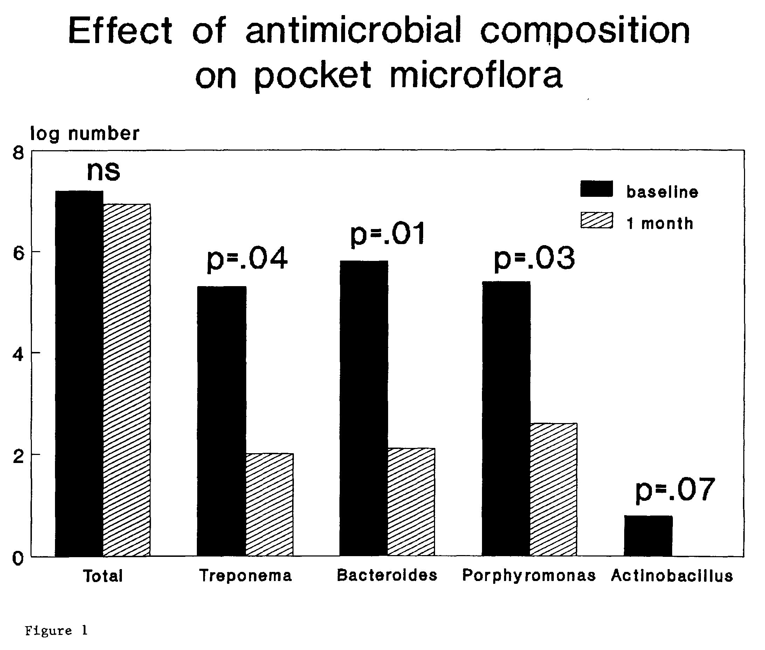

Compared with baseline levels, statistically significant reductions in all periodontal pathogens were observed after application of the antimicrobial composition, whereas no significant reductions were observed in the control group. This observation is illustrated by Figure 1.

Clinically, in the experimental group pocket depths were significantly reduced compared with the control pockets (P<.05).

It is concluded that even without supporting simultaneous mechanical treatment effective treatment of periodontal pathogens can be obtained by application of the antimicrobial composition. These positive results are even more surprising as only a single pocket was treated, and it is known that reinfection by periodontal pathogens is possible from other untreated periodontal pockets or mucosal surfaces.

### Example 2.

In this example the effect of the antimicrobial composition on the presence of Sulphate Reducing Bacteria, SRB, was investigated. SRB use sulphate as terminal electron acceptor to oxidize low-molecular-weight organic compounds, mainly products of microbial fermentation. Proteoglycans, the major component in the extracellular matrix of connective tissues represent an important source of sulphate. Therefore, SRB can be used as an indicator for breakdown of periodontal pockets.

In five refractory periodontal patients the effect of mechanical treatment was compared with application of the antimicrobial composition. When the antimicrobial composition was applied no adjunctive mechanical treatment was given. The result (Table 2) indicate that SRB are difficult to combat using mechanical treatment, but that application of the antimicrobial composition is highly effective.

**Table 2**

| isolation frequency of Sulphate Reducing Bacteria (SRB) in periodontal pockets | | |
|---|---|---|
| SRB positive pockets | mechanical treatment | antimicrobial composition |
| before | 8 | 10 |
| after 3 months | 7 | 1 |
| statistics | not significant | P<.05 |

It is concluded that after single application of the antimicrobial composition SRB can be eliminated, indicating arrestment of tissue breakdown in the periodontal pocket.

The antimicrobial composition can also be used to prevent, or in later stages, to treat the destructive inflammatory process around oral and non-oral osseo-intregated implants. This so called 'periimplantitis' is of great concern as the conventional mechanical treatment that is used for periodontal pockets cannot be used for implants. The composition of the bacterial microflora around implants is similar to that in periodontal pockets and, as we have shown above, the antimicrobial composition can be used effectively to combat this infection, even without combined mechanical treatment.

## Claims

1. A method for preventing or treating parodontitis in a mammal, wherein said mammal is treated with an antimicrobial composition comprising a physiologically acceptable varnish base and dissolved therein an antimicrobial agent.

2. A method for preventing diseases associated with infections induced by bacteria in periodontal pockets in a mammal, wherein said mammal is treated with an antimicrobial composition comprising a physiologically acceptable varnish base and dissolved therein an antimicrobial agent.

3. A method according to claim 1 or 2, wherein the mammal is a human.

4. A method according to any of the preceding claims, wherein the antimicrobial agent is chlorhexidine or a salt thereof, triclosan or a combination thereof.

5. A method according to any one of the preceding claims, wherein the varnish base is a natural resin dissolved in ethanol.

6. A method according to claim 5, wherein the resin is Sandarac, Siam or Sumatra benzoin, mastic, tolu balsam or a combination thereof.

7. A method according to any one of the preceding claims, wherein the composition further comprises an antibiotic, a quaternary ammonium compound, a plant alkaloid, a metal salt, a non-charged phenolic agent, a surface modifying agent, a phosphate compound, an oxidative enzyme, a bicarbonate, a peroxide, or a combination thereof.

8. A method according to claim 7, wherein the composition comprises cetyl pyridinium chloride, sanguinarine, a metal fluoride, zinc citrate, thymol, triclosan, delmopinol, an amyloglucosidase, a glucose oxidase, hydrogen peroxide, a bicarbonate salt, a (non-steroidal) anti-inflammatory agent, a protease inhibitor, a (poly-)calcium lactate, or a combination thereof.

9. A method according to any one of the preceding claims, wherein the composition is applied to a periodontal pocket.

10. Use of an antimicrobial composition comprising a physiologically acceptable varnish base and dissolved therein an antimicrobial agent for manufacturing a medicament for preventing or treating parodontitis.

11. Use of an antimicrobial composition comprising a physiologically acceptable varnish base and dissolved therein an antimicrobial agent for manufacturing a medicament for preventing diseases associated with infections induced by bacteria in periodontal pockets in a mammal.
